(19) Europäisches Patentamt
European Patent Office
Office européen des brevets

(11) **EP 3 818 930 B1**

(12) **EUROPEAN PATENT SPECIFICATION**

(45) Date of publication and mention
of the grant of the patent:
**12.03.2025 Bulletin 2025/11**

(21) Application number: **19830682.1**

(22) Date of filing: **04.07.2019**

(51) International Patent Classification (IPC):
*A61B 5/024* (2006.01)    *A61B 5/00* (2006.01)
*A61B 5/021* (2006.01)    *A61B 5/145* (2006.01)
*A61B 5/02* (2006.01)

(52) Cooperative Patent Classification (CPC):
**A61B 5/6897; A61B 5/00; A61B 5/02; A61B 5/021;
A61B 5/02108; A61B 5/024; A61B 5/02416;
A61B 5/145; A61B 5/681; A61B 5/6898;**
A61B 5/6843; A61B 5/7278

(86) International application number:
**PCT/KR2019/008215**

(87) International publication number:
**WO 2020/009496 (09.01.2020 Gazette 2020/02)**

(54) **BIOMETRIC INFORMATION MEASUREMENT DEVICE**

VORRICHTUNG ZUR MESSUNG BIOMETRISCHER INFORMATIONEN

DISPOSITIF DE MESURE D'INFORMATIONS BIOMÉTRIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.07.2018 KR 20180078921
26.06.2019 KR 20190076285**

(43) Date of publication of application:
**12.05.2021 Bulletin 2021/19**

(73) Proprietor: **Samsung Electronics Co., Ltd.
Suwon-si, Gyeonggi-do 16677 (KR)**

(72) Inventors:
• **HWANG, Jeong Eun
Suwon-si, Gyeonggi-do 16697 (KR)**
• **KO, Byung Hoon
Hwaseong-si, Gyeonggi-do 18476 (KR)**
• **CHOI, Chang Mok
Gyeonggi-do 16222 (KR)**
• **PARK, Sang Yun
Hwaseong-si, Gyeonggi-do 18436 (KR)**

(74) Representative: **Grünecker Patent- und
Rechtsanwälte
PartG mbB
Leopoldstraße 4
80802 München (DE)**

(56) References cited:
EP-A1- 3 069 658      WO-A2-2009/125349
JP-A- 2009 082 627      JP-A- 2009 082 627
KR-A- 20010 074 845      KR-A- 20180 045 383
KR-A- 20180 076 050      US-A1- 2002 095 092
US-A1- 2017 127 958

• **TENG X F ET AL: "The effect of contacting force
on photoplethysmographic signals; The effect of
contacting force on photoplethysmographic
signals", PHYSIOLOGICAL MEASUREMENT,
INSTITUTE OF PHYSICS PUBLISHING, BRISTOL,
GB, vol. 25, no. 5, 1 October 2004 (2004-10-01),
pages 1323 - 1335, XP020074200, ISSN:
0967-3334, DOI: 10.1088/0967-3334/25/5/020**

**Description**

**BACKGROUND**

**1. Field**

The following description relates to an apparatus and method for measuring bio-information.

**2. Description of Related Art**

A pressurized cuff-based method is used for general blood pressure measurement. The pressurized cuff-based method is a non-continuous measurement method in which a cuff is used to tighten blood vessels up to the maximum blood pressure and loosen to measure a blood pressure. The pressurized cuff-based method is not easy to apply to a portable device due to the configuration of a pressurizing pump or the like.

Recently, a blood pressure measurement apparatus employing non-pressurized cuffless method for measuring a blood pressure without using a cuff has been studied. For example, there are a pulse transit time (PTT)-based blood pressure measurement apparatus and a pulse wave analysis (PWA)-based blood pressure measurement apparatus. The PTT method is inconvenient in that it is necessary to perform correction for each individual for accurate measurement. Also, it is difficult to construct a compact device because bio-signals must be measured at two or more positions in order to measure the pulse wave velocity. On the other hand, the PWA method estimates a blood pressure only by analyzing the pulse wave form, and thus it is vulnerable to noise and has a limitation in accurate blood pressure measurement.
WO 2009/125349 A2 discloses a system for measuring blood circulation parameters in a patient, comprising: a) a pressing element; b) a contact microphone which is acoustically coupled to the patients body by the pressing element at a location adjacent to an artery, the microphone thereby producing a data signal indicative of blood circulation in the artery; and c) a controller which integrates in time the data signal from the microphone, thereby determining a blood pressure as a function of time, at least up to an unknown integration constant.
Teng X F et al: "The effect of contacting force on photoplethysmorgraphic signals; The effect of contacting force on photoplethysmorgraphic signals", Physiological Measurement, Institute of Physics Publishing Bristol, GB, vol. 25, no. 5, 1 October 2004 (2004-10-01), discloses the effect of contacting force on photoplethysmographic signals.
US 2017/127958 A1 discloses a photoplethysmographic device for measuring a heart rhythm, comprising a housing having a contacting surface and a control unit, a photoplethysmographic sensor mechanically attached to the contacting surface and connected to the control unit, wherein the control unit is arranged to receive a photoplethysmographic signal from the photoplethysmographic sensor, a fastening band having a first end and a second end, the first end and second end attached to the device for attaching the device to a subject with the contacting surface contacting the subject's skin, a pressure sensor attached to the contacting surface in close proximity to the photoplethysmographic sensor. The control unit is arranged determining whether the pressure from the pressure sensor is in a range suitable for photoplethysmographic measurement by the photoplethysmographic sensor.

**SUMMARY**

It is therefore the object of the present invention to provide an improved apparatus for measuring bio-information and a corresponding method of measuring bio-information.
This object is solved by the subject matter of the independent claim. Preferred embodiments are defined by the dependent claims.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

The following description relates to an apparatus and method for measuring bio-information.

In one general aspect, an apparatus for measuring bio-information includes a pulse wave sensor configured to have a curved contact surface, convex toward a contact surface of an object of interest, in contact with the object of interest and measure one or more pulse wave signals from the object in contact with the contact surface, a force sensor disposed below or on a side of the pulse wave sensor and configured to measure a contact force of the object, and a processor configured to estimate bio-information of the object on the basis of the one or more measured pulse wave signals and the measured contact force.

The pulse wave sensor may include a housing having the curved contact surface and a pulse wave measurer mounted in the housing and configured to measure the one or more pulse wave signals from the object in contact with the contact surface.

The housing is formed in a semi-cylindrical shape or a hemi-ellipsoid shape.

The housing may be formed to have a size smaller than a size of a finger.

The housing may be formed to have a size smaller than an average finger size of a plurality of users.

The housing has a first radius of curvature $R_1$ which is not less than 2 mm and not more than 10 mm and a second radius of curvature $R_2$ which is not less than $0.5 * R_1$ and not more than $4 * R_1$.

The housing may have a surface roughness of 1.6 $\mu$m or less.

The housing may have a stiffness of 0.5 GPa or greater.

The housing is formed as a semi-cylindrical or hemi-ellipsoid shape which has a length greater than zero and equal to or less than 16 mm.

The pulse wave signals may be photoplethysmogram (PPG) signals.

The pulse wave measurer may include one or more light sources configured to emit light to the object of interest and a photodetector configured to receive light returning from the object and measure the one or more pulse wave signals.

The processor may acquire an oscillometric waveform using the one or more pulse wave signals and the contact force and estimate the bio-information by analyzing a change in oscillometric waveform.

The processor may select one or more pulse wave signals from among the one or more pulse wave signals and acquire the oscillometric waveform using the one or more selected pulse wave signals and the contact force.

The processor may select the one or more pulse wave signals on the basis of at least one of a maximum amplitude value of each of the pulse wave signals, an average amplitude value, and a difference between a maximum amplitude value and a minimum amplitude value of each of the pulse wave signals.

The processor may generate and provide contact pressure guide information on the basis of the measured contact force during the measurement of the plurality of pulse wave signals.

The bio-information may be blood pressure.

The apparatus for measuring bio-information may not include a contact area sensor that measures a contact area with the object of interest.

In another general aspect, a method of measuring bio-information includes measuring one or more pulse wave signals from an object of interest in contact with a contact surface of a pulse wave sensor which is a curved surface, convex toward a contact surface of the object of interest, measuring a contact force between the pulse wave sensor and the object; and estimating bio-information of the object on the basis of the one or more measured pulse wave signals and the measured contact force.

The pulse wave signals may be PPG signals.

The measuring of the one or more pulse wave signals may include emitting light to the object and measuring the one or more pulse wave signals by receiving light returning from the object.

The estimating of the bio-information may include acquiring an oscillometric waveform using the one or more pulse wave signals and the contact force and estimating the bio-information by analyzing a change in oscillometric waveform.

The acquiring of the oscillometric waveform may include selecting one or more pulse wave signals from among the one or more pulse wave signals and acquiring the oscillometric waveform using the one or more selected pulse wave signals and the contact force.

The selecting of the one or more pulse wave signals may include selecting the one or more pulse wave signals on the basis of at least one of a maximum amplitude value of each of the pulse wave signals, an average amplitude value, and a difference between a maximum amplitude value and a minimum amplitude value of each of the pulse wave signals.

The method may further include generating and providing contact pressure guide information on the basis of the measured contact force during the measurement of the plurality of pulse wave signals.

The bio-information may be blood pressure.

Other features and aspects will be apparent from the following detailed description, the drawings, and the claims.

## BRIEF DESCRIPTION OF THE DRAWINGS

FIG. 1 is a diagram illustrating an apparatus for measuring bio-information according to one embodiment of the present disclosure.

FIG. 2 is a diagram illustrating one example of a structure of a housing.

FIG. 3 is a diagram illustrating another example of a structure of the housing.

FIG. 4 is a diagram illustrating still another example of a structure of the housing (not according to the invention).

FIG. 5 is a diagram for describing a size of the housing.

FIG. 6A is a diagram for describing a relationship between a contact pressure and a contact force when the pulse wave sensor is formed in a hemispherical shape.

FIG. 6B is a diagram for describing a relationship between a contact pressure and a contact force when the pulse wave sensor is formed in a semi-cylindrical shape.

FIG. 7A illustrates graphs showing an example of an oscillometric waveform.

FIG. 7B illustrates graphs showing examples of a pulse wave signal of an oscillometric waveform and a contact force.

FIG. 7C illustrates graphs on which data on contact force values and diastolic blood pressure values acquired from a plurality of objects of interest are plotted as XY coordinates.

FIG. 7D is a graph on which data on contact force values and systolic blood pressure values acquired from a plurality of objects of interest are plotted as XY coordinates.

FIG. 8A is a diagram illustrating one example of a pulse wave measurer.

FIG. 8B is a diagram illustrating another example of the pulse wave measurer.

FIG. 8C is a diagram illustrating still another example of the pulse wave measurer.

FIG. 9A is a diagram illustrating an example of an arrangement of light sources and a photodetector when a pulse wave sensor is formed in a semi-cylindrical shape.

FIG. 9B is a diagram illustrating an example of an arrangement of light sources and a photodetector when a pulse wave sensor is formed in a hemi-ellipsoid shape.

FIG. 9C is a diagram illustrating an example of an arrangement of a light source and a photodetector when a pulse wave sensor is formed in a hemispherical shape.

FIG. 10 is a diagram illustrating an apparatus for measuring bio-information according to another embodiment of the present disclosure.

FIG. 11 is a diagram illustrating an example to which an apparatus for measuring bio-information is applied.

FIG. 12 is a diagram illustrating another example to which an apparatus for measuring bio-information is applied.

FIG. 13 is a diagram illustrating still another example to which an apparatus for measuring bio-information is applied.

FIG. 14 is a diagram illustrating yet another example to which an apparatus for measuring bio-information is applied.

FIG. 15 is a diagram illustrating still another example to which an apparatus for measuring bio-information is applied.

FIG. 16 is a diagram illustrating another example to which an apparatus for measuring bio-information is applied.

FIG. 17 is a diagram illustrating another example to which an apparatus for measuring bio-information is applied.

FIG. 18 is a flowchart illustrating a method of measuring bio-information according to one embodiment of the present disclosure.

Throughout the drawings and the detailed description, unless otherwise described, the same drawing reference numerals will be understood to refer to the same elements, features, and structures. The relative size and depiction of these elements may be exaggerated for clarity, illustration, and convenience.

## DETAILED DESCRIPTION

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters.

It should be noted that in some alternative implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved.

As used herein, the singular forms are intended to include the plural forms as well, unless the context clearly indicates otherwise. It will be further understood that the terms "comprises" and/or "comprising," or "includes" and/or "including" when used in this description, specify the presence of stated features, numbers, steps, operations, elements, components or combinations thereof, but do not preclude the presence or addition of one or more other features, numbers, steps, operations, elements, components or combinations thereof.

It will also be understood that the elements or components in the following description are discriminated in accordance with their respective main functions. In other words, two or more elements may be made into one element or one element may be divided into two or more elements in accordance with a subdivided function. Additionally, each of the elements in the following description may perform a part or whole of the function of another element as well as its main function, and some of the main functions of each of the elements may be performed exclusively by other elements. Each element may be realized in the form of a hardware component, a software component, and/or a combination thereof.

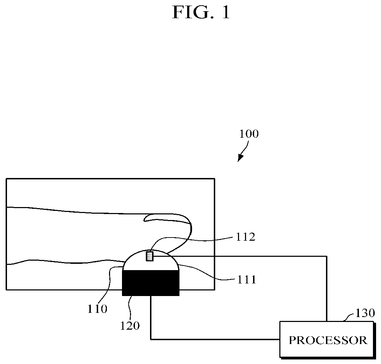

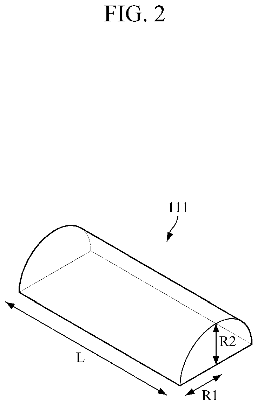

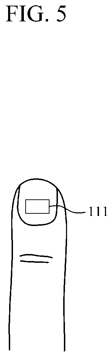

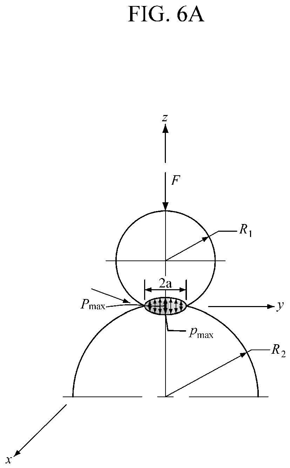

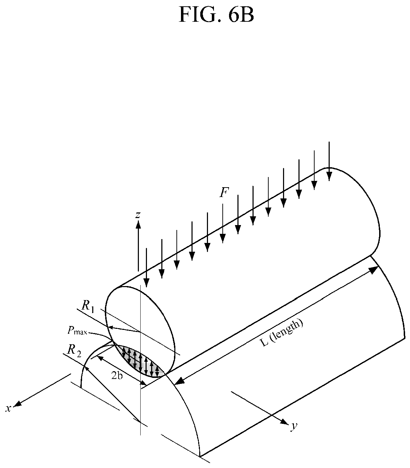

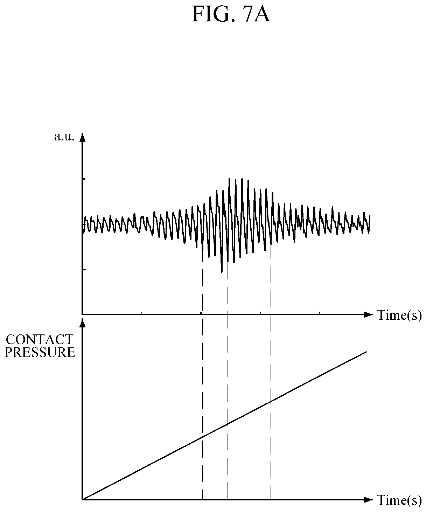

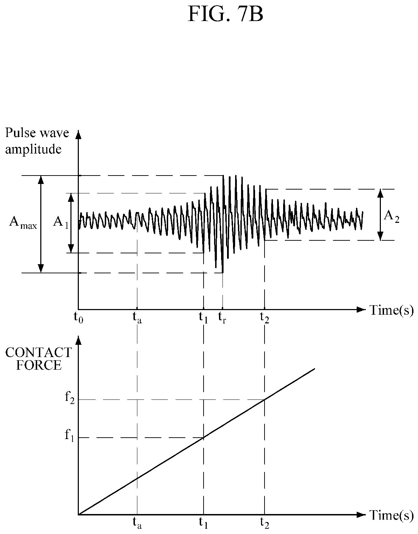

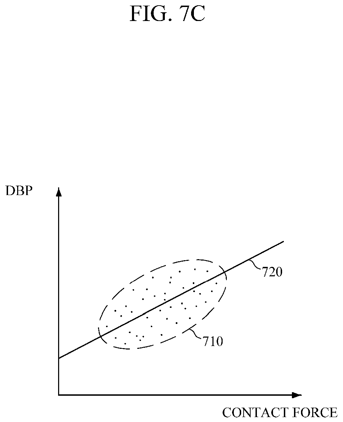

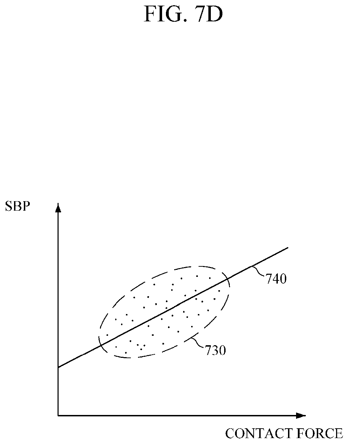

FIG. 1 is a diagram illustrating an apparatus for measuring bio-information according to one embodiment of the present disclosure. FIGS. 2 to 4 are diagrams illustrating examples of a structure of a housing. FIG. 5 is a diagram for describing a size of the housing and FIGS. 6 and 6B are diagrams for describing a relationship between a contact pressure and a contact force in accordance with a structure of a pulse wave sensor. FIG. 7A illustrates graphs showing an example of an oscillometric waveform, FIG. 7B illustrates graphs showing examples of a pulse wave signal of an oscillometric waveform and a contact force, FIG. 7C illustrates graphs on which data on contact force values and diastolic blood pressure values acquired from a plurality of objects of interest are plotted as XY coordinates, and FIG. 7D is a graph on which data on contact force values and systolic blood pressure values acquired from a plurality of objects of interest are plotted as XY coordinates. The apparatus 100 for measuring bio-information shown in FIG. 1 may be mounted in an electronic device, an accessory of an electronic device (e.g., a protective case of an electronic device or the like), a stylus pen, or the like. In this case, the electronic device may include a mobile phone, a smartphone, a tablet terminal, a notebook computer, a personal digital assistant (PDA), a portable multimedia player (PMP), a navigation device, an MP3 player, a digital camera, a wearable device, and the like, and the wearable device may include various types of wearable devices, such as a wristwatch type, a wrist-band type, a belt type, a necklace type, an ankle band type, a thigh band type, a forearm band type, and the like. However, the electronic device is not limited to the above examples and the wearable device is also not limited to the above examples.

Referring to FIG. 1, the apparatus 100 includes a pulse wave sensor 100, a force sensor 120, and a processor 130.

The pulse wave sensor 110 measure one or a plurality of pulse wave signals from the object in contact with the contact surface. Here, the pulse wave signal may be a photoplethysmogram (PPG) signal. When the pulse wave sensor 110 measures a plurality of pulse wave signals, the plurality of pulse wave signals may be measured using light of different wavelengths. Here, the object of interest may be an area of the human body which can be in contact with the pulse wave sensor 110 and from which a pulse wave signal can be easily measured. For example, the object may be a peripheral part of the body, such as a finger, a toe, or the like, or a region of a wrist surface adjacent to the radial artery which is an upper area of the wrist through which capillary blood or venous blood passes. Hereinafter, for convenience of description, a finger will be described as an example of an object of interest.

The elasticity of a finger is affected by the stiffness and structure of a contact object. For example, as compared to a contact object having a flat surface, a contact object having a curved surface deforms a deeper layer of the finger when the same force is exerted. Therefore, the pulse wave sensor 110 according to one embodiment has a curved contact surface in contact with the finger so that the same pressure can be exerted on the finger with a less force than applied to a flat contact surface.

The pulse wave sensor 110 may include a housing 111 and a pulse wave measurer 112.

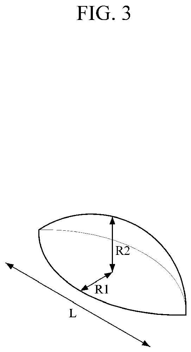

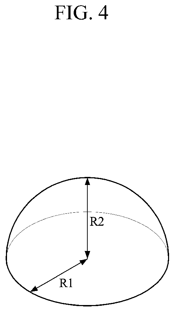

The housing 111 has a curved contact surface, convex to a contact surface of the finger, in contact with the finger in consideration of the elasticity and anatomical structure of the finger. The housing 111 is formed in a semi-cylindrical shape as shown in FIG. 2, or a hemi-ellipsoid shape as shown in FIG. 3, or a hemispherical shape as shown in FIG. 4 (not according to the invention). In this case, the housing 111 may be formed to have a smaller size than the finger as shown in FIG. 5, so that the contact area between the finger and the housing 111 can be constant. For example, the housing 111 may be formed to have a size smaller than an average size of fingers of a plurality of users by taking into consideration the age and sex of users, a type of finger to be used (e.g., a thumb, an index finger, a middle finger, a ring finger, or a little finger), and the like. The housing 111 is formed to have a first radius of curvature R1 which is not less than 2 mm and not more than 10 mm and a second radius of curvature R2 which is not less than 0.5 * R1 and not more than 4 * R1, in consideration of the maximum width/thickness of the finger and/or the size of the pulse wave sensor 110. In addition, a length L of the housing 111 is greater than 0 mm and equal to or less than 16 mm in consideration of the width of the finger.

The structure of the housing enables a pressure to be well delivered into the finger with less force than applied to a structure of a flat surface, and thus it is possible to easily reach a maximum pulse pressure when blood pressure is measured using the oscillometric technique. In addition, the apparatus 100 for measuring bio-information may easily acquire information of the inside of the finger (e.g., a blood vessel and blood under the skin) through the above-described housing structure which allows the apparatus 100 to be located accurately, close to a target (e.g., a blood vessel or the like) from which the bio-information is to be obtained.

A force of the finger generates a frictional force due to the anatomical structure of finger bones and adhesive properties and elasticity of the finger skin when the finger is in contact with an object. Because the frictional force varies according to the shape and material of the surface in contact with the finger, even when a predetermined pressure is applied to the finger, a loss due to a frictional force with the contact object is generated so that the pressure externally applied to the finger may not be entirely transmitted to the inside of the finger. Therefore, the material and/or the structure of the housing **111** may need to be appropriately selected such that the influence of the frictional force can be minimized.

When the stiffness of the contact object is lower than or similar to that of the finger, the contact object deforms together with the finger when the finger applies a force to the contact object, and thus the force causing the deformation of the contact object may be lost and may not be transmitted to the finger. Therefore, the stiffness of the housing 111 should be much larger than that of the finger such that the housing 111 is free from deformation from the force applied. In addition, the housing 111 may be formed of a material stronger than rubber or polyethylene. For example, the housing 111 may be formed of a material with a stiffness of 0.5 GPa or greater (e.g., carbon fiber, high strength plastic, metal, etc.).

Because a contact object having a high surface roughness has a large contact friction and adhesion when in contact with the finger, the housing 111 may be formed of a material having a smooth surface with a roughness lower than a predetermined level. For example, the housing 111 may be formed of a material with a surface roughness of 1.6 $\mu$m or less.

The pulse wave measurer 112 may be mounted in the housing 111 and measure one or a plurality of pulse wave signals from the finger in contact with the curved surface of the housing 111. When the pulse wave measurer 112 measures a plurality of pulse wave signals, the plurality of pulse wave signals may be measured using light of different wavelengths. According to one embodiment, the pulse wave measurer 112 may include one or a plurality of light sources configured to emit light to the object of interest in contact with the curved surface of the housing 111 and a photodetector configured to receive light returning from the object of interest.

The force sensor 120 may measure a contact force between the object of interest and the pulse wave sensor 110. The force sensor 120 may be disposed below or on a side of the pulse wave sensor 110, but is not limited thereto. According to one embodiment, the force sensor 120 may measure a force exerted on the force sensor 120 due to the contact between the object of interest and the pulse wave sensor 110. According to one embodiment, the force sensor 120 may include a resistive force sensor, an ultrasonic force sensor, a load cell sensor, a capacitive force sensor, a pyroelectric force sensor, a strain gage force sensor, an electrochemical force sensor, an optical force sensor, a magnetic force sensor, and the like.

FIGS. 6A to 6B are diagrams for describing a relationship between a contact pressure and a contact force in accordance with a structure of a pulse wave sensor. More specifically, FIG. 6A is a diagram for describing a relationship between a contact pressure and a contact force when the pulse wave sensor is formed in a hemispherical shape, and FIG. 6B is a diagram for describing a relationship between a contact pressure and a contact force when the pulse wave sensor is formed in a semi-cylindrical shape.

Referring to FIG. 6A, when a sphere having a radius of $R_1$ is in contact with a sphere having a radius of $R_2$, a contact area may appear as a circle having a radius of a. In this case, the radius a of the contact area may be represented as Equation 1 and a maximum contact pressure $P_{max}$ appearing at the center of the contact area may be represented as Equation 2.

$$a = \sqrt[3]{\frac{3F\left[\frac{1-v_1^2}{E_1} + \frac{1-v_2^2}{E_2}\right]}{4\left(\frac{1}{R_1} + \frac{1}{R_2}\right)}}$$

$$\ldots(1)$$

$$P_{max} = \frac{3F}{2\pi a^2} \qquad \ldots(2)$$

Here, $E_1$ and $E_2$ may denote a modulus of elasticity of a sphere having a radius of $R_1$ and a modulus of elasticity of a sphere having a radius of $R_2$, respectively, $v_1$ and $v_2$ may denote a Poisson's ratio of the sphere having a radius of $R_1$ and a Poisson's ratio of the sphere having a radius of $R_2$, respectively, and $F$ may denote a force externally exerted to the sphere having a radius of $R_1$.

As shown in Equation 2, when the sphere having a radius of $R_1$ is in contact with the sphere having a radius of $R_2$, the maximum contact pressure $P_{max}$ of the two objects may be determined by the contact area $2\pi a^2$ and the force F externally exerted on the sphere having a radius of $R_1$. Therefore, when the housing 111 is realized to have a size smaller than the size of the finger, it is possible to have a fixed contact area $2\pi a^2$ irrespective of the size of the finger and it is possible to calculate the contact pressure between an object of interest and the housing 111 only with the force sensor 120 without requiring a separate contact area sensor.

Referring to FIG. 6B, when a cylinder having a radius of $R_1$ is in contact with a semi-cylinder having a radius of $R_2$, the contact area may appear as a rectangle having a width of 2b and a length of L. In this case, a half-width b of the contact area may be represented as Equation 3 and the maximum contact pressure $P_{max}$ appearing at the center line of the contact area may be represented as Equation 4.

$$b = \sqrt{\frac{4F\left[\dfrac{1 - v_1^2}{E_1} + \dfrac{1 - v_2^2}{E_2}\right]}{\pi L\left(\dfrac{1}{R_1} + \dfrac{1}{R_2}\right)}} \qquad \ldots(3)$$

$$P_{max} = \frac{2F}{\pi 2 b L} \qquad \ldots(4)$$

Here, $E_1$ and $E_2$ may denote a modulus of elasticity of a cylinder having a radius of $R_1$ and a modulus of elasticity of a semi-cylinder having a radius of $R_2$, respectively, $v_1$ and $v_2$ may denote a Poisson's ratio of the cylinder having a radius of $R_1$ and a Poisson's ratio of the semi-cylinder having a radius of $R_2$, respectively, $F$ may denote a force externally exerted to the cylinder having a radius of $R_1$, and L may denote a contact length.

As shown in Equation 4, when the cylinder having a radius of $R_1$ is in contact with the semi-cylinder having a radius of $R_2$, the maximum contact pressure $P_{max}$ of the two objects may be determined by the contact area $\pi bL$ and the force F externally applied to the cylinder having a radius of $R_1$. Thus, when the housing 111 is realized to have a size smaller than the size of the object of interest (e.g., a finger), it is possible to have a fixed contact area $\pi bL$ regardless of the size of the object of interest (e.g., a finger) and it is possible to caclulae the contact pressure between the object of interest and the housing 111 with only the force sensor 120 without requiring a separate contact area sensor.

The processor 130 may control the overall operation of the apparatus 100 for measuring bio-information.

The processor 130 may control the pulse wave sensor 110 to measure one or a plurality of pulse wave signals necessary for measuring bio-information. When a request for measuring bio-information is received from a user, the processor 130 may generate a pulse wave sensor control signal to control the pulse wave sensor 110. Conditions for driving the pulse wave sensor may be stored in a storage device in advance. When the request for measuring bio-information is received, the processor 130 may control the pulse wave sensor 110 by referring to the sensor driving conditions stored in the storage device. In this case, the sensor driving conditions may include an emission time of each light source, an order of driving, a current intensity, pulse duration, and the like.

The processor 130 may generate contact pressure guide information for guiding the user to increase or decrease a pressure applied to the pulse wave sensor 110 during the measurement of the pulse wave signal and provide the contact pressure guide information to the user. The processor 130 may provide the contact pressure guide information by visually displaying it or using a non-visual method, such as voice or vibration. The contact pressure may be calculated from the value measured by the force sensor 120 as described above.

The contact pressure guide information may be provided before, after, or simultaneously with the starting of the pulse wave signal measurement by the pulse wave sensor 110. The contact pressure guide information may be continuously provided to the user while the pulse wave sensor 110 measures the pulse wave signals from the object of interest. The contact pressure guide information may be set for each user in advance on the basis of user characteristics, such as the age, the sex, a health status, a contact area of an object, and the like of the user. The contact pressure guide information may be a pressure value to be increased or decreased in the pulse wave sensor 110 by the user, but is not limited thereto, and may include action information of the user to guide a change in pressure applied to the pulse wave sensor 110 by the object of interest.

When the request for measuring bio-information is received, the processor 130 may generate a control signal to control the force sensor 120 to measure a contact force.

The processor 130 may continuously receive a contact force measurement value from the force sensor 120, calculate a contact pressure value on the basis of the received contact force measurement value, and generate and provide contact pressure guide information using the calculated contact pressure value. For example, the processor 130 may provide the contact pressure guide information on the basis of a difference between the contact pressure value obtained at a specific instance and a contact pressure value to be applied to the pulse wave sensor 110 by the user at the specific instance.

The processor 130 may acquire an oscillometric waveform using the one or the plurality of pulse wave signals acquired through the pulse wave sensor 110 and the contact force acquired through the force sensor 120. In this case, the oscillometric waveform may represent a change of the pulse wave signal in accordance with the change of the contact pressure as shown in FIG. 7A.

According to one embodiment, the processor 130 may select one or a plurality of pulse wave signals from among the pulse wave signals acquired from the pulse wave sensor 110 according to predetermined criteria and acquire the oscillometric waveform using a combination of the selected pulse wave signals and the contact pressure calculated based on the contact force acquired from the force sensor 120. In this case, the predetermined criteria may include at least one of the maximum amplitude value of each of the pulse wave signals, an average amplitude value, and a difference between the maximum amplitude value and the minimum amplitude value of each of the pulse wave signals. However, the embodiment is not limited thereto and a pulse wave signal measured using light of a predetermined wavelength may be selected and used from among the acquired pulse wave signals. For example, the processor 130 may select one pulse wave signal having the greatest difference between the maximum amplitude value and the minimum amplitude value and acquire the oscillometric waveform using the selected pulse wave signal and the contact pressure.

The processor 130 may estimate bio-information by analyzing a change in oscillometric waveform in accordance with a change in contact pressure. In this case, the bio-information may include, but not limited to, blood pressure, blood sugar level, cholesterol level, vascular age, arterial stiffness, aortic artery pressure waveform, stress index, fatigue level, and the like. However, for convenience of description, a blood pressure will be hereinafter described as an example of the bio-information.

A blood pressure may include diastolic blood pressure (DBP), systolic blood pressure (SBP), and mean arterial pressure (MAP), and a contact pressure applied to the finger may act as an external pressure applied to a blood vessel. When the contact pressure is smaller than the MAP, the elastic restoring force of tissues acts in a direction of compressing the blood vessel and thereby the amplitude of the oscillometric waveform becomes smaller. When the contact pressure is equal to the MAP, the elastic restoring force of tissues becomes zero and thus does not affect the blood vessel so that the amplitude of the oscillometric waveform is maximized. In addition, when the contact pressure is greater than the MAP, the elastic restoring force of tissues acts in a direction of expanding the blood vessel, and thereby the amplitude of the oscillometric waveform becomes smaller. Accordingly, the processor 130 may analyze the change in oscillometric waveform in accordance with the contact pressure and estimate the MAP using the contact pressure at a point where the amplitude of the oscillometric waveform is maximized. In addition, the processor 130 may estimate the DBP using a contact pressure at a point where an amplitude corresponds to a first proportion (e.g., 0.7) relative to the maximum amplitude of the oscillometric waveform and estimate the SBP using a contact pressure at a point where an amplitude corresponds to a second proportion (e.g., 0.6) relative to the maximum amplitude of the oscillometric waveform. In this case, a correlation between the MAP and the contact pressure when the amplitude of the oscillometric waveform is maximum, a correlation between the SBP and the contact pressure at the point where the amplitude is the first proportion relative to the maximum amplitude, and a correlation between the DBP and the contact pressure at the point where the amplitude is the second proportion relative to the maximum amplitude may be pre-defined through experiments.

In order to measure blood pressure, the user may bring his/her finger into contact with the housing 111 of the apparatus 100 for measuring bio-information and then gradually increase a force applied to the housing 111. In this case, the pulse wave sensor 110 of the apparatus 100 may output a pulse wave signal in an oscillometric waveform as shown in the upper part of FIG. 7B and the force sensor 120 may output a contact force signal which increases over time as shown in the lower part of FIG. 7B.

When the apparatus 100 with the above-described structure for measuring bio-information is used, at an initial stage in which the user brings his/her finger into contact with the housing 111 for the first time and increases force, a change in contact area between the finger and the sensor may occur. However, the change in contact area is negligible or zero during a time period for which pulse wave information that is meaningful to estimate blood pressure is acquired. Therefore, when blood pressure is measured through the apparatus 100 for measuring bio-information, it may be considered that the contact area between the finger of the user and the housing 111 is fixed. For example, in the pulse wave signal shown in the upper part of FIG. 7B, during an interval from a time point $t_0$ at which the user brings his/her finger into contact with the housing 111 for the first time to a time point $t_a$ at which a contact force is somewhat increased, the contact area between the finger and the housing 111 may be increased. However, the contact area is rarely changed in the interval after a time point $t_a$ and the pulse wave signal necessary for blood pressure estimation may be included in this interval.

Therefore, the processor 130 does not calculate a contact pressure value, but estimate blood pressure of the user using a blood pressure estimation function in which the contact force value acquired from the force sensor 120 is taken as an input parameter. The blood pressure estimation function may be stored in an internal or external memory of the processor 130, and there may be a DBP estimation function and an SBP estimation function independently of each other. The blood pressure estimation function may be obtained in advance from experiments on a plurality of objects of interest.

Hereinafter, a method of acquiring a blood pressure estimation function will be described in detail.

Pulse wave signals in an oscillometric waveform and contact force signals may be acquired from a plurality of objects of interest using the apparatus for estimating bio-information, which has the structure described with reference to FIGS. 1 to 5. The pulse wave signals and the contact force signals acquired from each object of interest may have forms similar to those shown in FIG. 7B. In addition, DBPs and SBPs of the objects of interest may be measured using a separate blood pressure measurement device, such as a cuff blood pressure monitor. In this case, blood pressures of the objects of interest may be measured at a time point at which there is no significant difference from the actual blood pressures of the objects of interest at the time of measuring the pulse wave signals and the contact force signals using the apparatus for measuring bio-information. For example, the blood pressures of the objects of interest may be measured during the measurement of the pulse wave signals and the contact force signals of the object of interest using the apparatus for measuring bio-information. Alternatively, the blood pressures of the objects of interest may be measured immediately before or immediately after the pulse wave signals and the contact force signals of the objects of interest are measured using the apparatus for measuring bio-information.

A blood pressure estimation function may be deduced using the pulse wave signals, contact force signals, and blood pressure values acquired through the above-described procedures. For example, it is assumed that a pulse wave signal in an oscillometric waveform as shown in the upper part of FIG. 7B is acquired. A time point $t_1$ may be selected at which a pulse wave having an amplitude corresponding to a first proportion relative to the maximum amplitude $A_{max}$ appears among pulse waves shown in the left side of a graph with respect to time point $t_r$ at which the amplitude of the pulse wave signal is maximum. A contact force value $f_1$ acquired through the force sensor may be acquired at the selected time point $t_1$. The acquired contact force value $f_1$ and a measured DBP value of a corresponding object of interest may be mapped and stored. The above-described procedures may be performed for the plurality of objects so that a plurality of contact force values and DBP values corresponding to the respective contact force values can be acquired.

FIG. 7C illustrates graphs on which data on contact force values and DBP values acquired from a plurality of objects of interest are plotted as XY coordinates. A DBP candidate function 720 may be acquired through regression analysis of this data set 710. More specifically, a relation between a contact force and a DBP may be derived through regression analysis in which the contact force values of the data set 710 are taken as independent variables and the DBP values are taken as dependent variables, and the relation may be used as the DBP candidate function 720. In this case, other mathematical techniques besides regression analysis may be used. A first proportion used to acquire the data set 710 may be used as a condition for acquiring a contact force value, which is an input parameter, when the DBP candidate function 720 is used as the DBP estimation function.

When the first proportion is changed in FIG. 7B, the contact force value $f_1$ may be changed. If the data set 710 in FIG. 7C is acquired by setting the first proportion to $X_1$, other data sets consisting of changed contact force values and corresponding DBP values may be acquired by adjusting the first proportion to $X_2$, $X_3$, and the like. DBP candidate functions for each of a plurality of data sets may be derived and each of the DBP candidate functions may output a DBP value expected when a contact force value of the data set is taken as an input. An average error between DBP values acquired through each DBP candidate function and the actual DBP value included in the data set may be calculated and a DBP candidate function with the smallest average error may be selected and used as the DBP estimation function.

A finally decided DBP estimation function and the first proportion corresponding to the DBP estimation function may be stored in the internal or external memory of the processor 130 of the apparatus 100 for measuring bio-information and be used when the processor 130 calculates a DBP of the user.

An example of the DBP estimation function acquired through the above-described procedures may be expressed by Equation 5.

$$BP_{DBP}(f_n) = af_n + b \qquad \ldots(5)$$

Even when DBP is estimated using Equation 5, the user may bring his/her finger into contact with the apparatus 100 for measuring bio-information and then gradually increase a pressing force. A pulse wave signal in an oscillometric waveform and a contact force signal which are accordingly have forms smilar to those shown in FIG. 7B. If a signal as shown in FIG. 7B is acquired, a contact force value $f_1$ at a time point $t_1$ at which a pulse wave having an amplitude $A_1$ corresponding to a first proportion $A_1$ relative to the maximum amplitude $A_{max}$ appears among pulse waves shown in the left side of the graph with respect to a time point $t_r$ at which the amplitude of the pulse wave signal is maximum may correspond to $f_n$ in Equation 5. In Equation 5, a and b are constants and may be determined according to characteristics of a sensor to be used or characteristics of a population of objects of interest.

In a similar manner as the above-described DBP estimation function, a SBP estimation function may be acquired. Referring to FIG. 7B, from a measured pulse wave signal in an oscillometric waveform of one object of interest, a time point $t_2$ may be selected at which a pulse wave having an amplitude corresponding to a second proportion $A_2$ relative to the maximum amplitude $A_{max}$ appears among pulse waves shown in the left side of the graph with respect to the time point $t_r$ at which the amplitude of the pulse wave signal is maximum. A contact force value f2 acquired through the force sensor at the selected time point t2 may be acquired. The acquired contact force value $f_2$ and a previously measured SBP value of the corresponding object of interest may be mapped and stored. The above procedures may be performed for each of a plurality of objects of interest so that a plurality of contact force values and SBP values corresponding to each of the contact force values can be acquired.

FIG. 7D is a graph on which data on contact force values and SBP values acquired from a plurality of objects of interest are plotted as XY coordinates. A DBP candidate function 740 may be acquired through regression analysis of this data set 730. More specifically, a relation between a contact force and a DBP may be derived through regression analysis in which the contact force values of the data set 730 are taken as independent variables and the SBP values are taken as dependent variables, and the relation may be used as the SBP candidate function 740. In this case, other mathematical techniques besides regression analysis may be used. A second proportion used to acquire the data set 730 may be used as a condition for acquiring a contact force value, which is an input parameter, when the SBP candidate function 740 is used as the SBP estimation function.

When the second proportion is changed in FIG. 7B, the contact force value $f_2$ may be changed. If the data set 730 in FIG. 7D is acquired by setting the second proportion to $Y_1$, other data sets consisting of changed contact force values and corresponding SBP values may be acquired by adjusting the second proportion to $Y_2$, $Y_3$, and the like. SBP candidate functions for each of a plurality of data sets may be derived and each of the SBP candidate functions may output an SBP value expected when a contact force value of the data set is taken as an input. An average error between SBP values acquired through each SBP candidate function and the actual SBP value included in the data set may be calculated and an SBP candidate function with the smallest average error may be selected and used as the SBP estimation function.

A finally decided SBP estimation function and the second proportion corresponding to the SBP estimation function may be stored in the internal or external memory of the processor 130 of the apparatus 100 for measuring bio-information and be used when the processor 130 calculates an SBP of the user.

An example of the SBP estimation function acquired through the above-described procedures may be expressed by Equation 6.

$$BP_{SBP}(f_m) = c\,f_m + d \qquad \ldots (6)$$

Even when SBP is estimated using Equation 6, the user may bring his/her finger into contact with the apparatus 100 for measruing bio-information and then gradually increase a pressing force. A pulse wave signal in an oscillometric waveform and a contact force signal which are accordingly acquired have forms smilar to those shown in FIG. 7B. If a signal as shown in FIG. 7B is acquired, a contact force value $f_2$ at a time point $t_2$ at which a pulse wave having an amplitude $A_2$ corresponding to a second proportion $A_2$ relative to the maximum amplitude $A_{max}$ appears among pulse waves shown in the left side of the graph with respect to a time point $t_r$ at which the amplitude of the pulse wave signal is maximum may correspond to $f_m$ in Equation 6. In Equation 6, c and d are constants and may be determined according to characteristics of a sensor to be used or characteristics of a population of objects of interest.

In the foregoing description, the DBP estimation function and the SBP estimation function are each described as a linear function, which is, however, merely an example. The blood pressure estimation function may be multi-order function, or may be a different type of function. In addition, a look-up table consisting of contact force values and estimated blood pressure values may be used instead of a function.

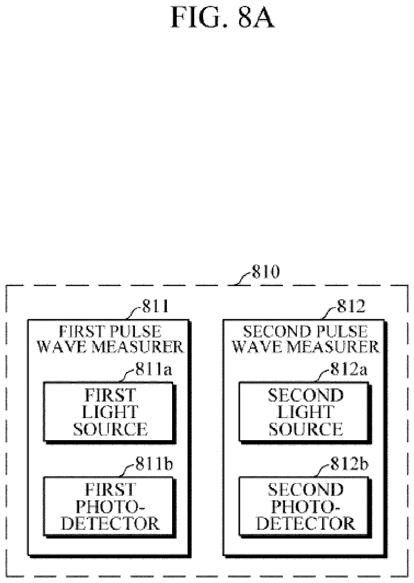

FIGS. 8A to 8C are diagrams illustrating examples of a pulse wave measurer. FIGS. 8A to 8C may illustrate examples of the pulse wave measurer 112 of FIG. 1. Various embodiments of the configuration of the pulse wave measurer which measures a plurality of pulse wave signals from an object of interest will be hereinafter described with reference to FIGS. 8A to 8C.

Referring to FIG. 8A, the pulse wave measurer 810 according to one embodiment may be formed as an array of pulse wave measurers to measure a plurality of pulse wave signals. As shown in FIG. 8A, the pulse wave measurer 810 may include a first pulse wave measurer 811 and a second pulse wave measurer 812. However, the number of pulse wave measurers constituting the array of pulse wave measurers is not particularly limited.

The first pulse wave measurer 811 may include a first light source 811a to emit light of a first wavelength to an object of interest. In addition, the first pulse wave measurer 811 may include a first photodetector 811b to receive light of the first wavelength returning from the object irradiated by the first light source 811a and measure a first pulse wave signal.

The second pulse wave measurer 812 may include a second light source 812a to emit light of a second wavelength to the object of interest. In addition, the second pulse wave measurer 812 may include a second photodetector 812b to receive light of the second wavelength returning from the object irradiated by the second light source 812a and measure a second pulse wave signal. In this case, the first wavelength may be different from the second wavelength.

Here, the first light source 811a and the second light source 812a may include a lightemitting diode (LED), a laser diode, and a phosphor, but are not limited thereto. In addition, the first photodetector 811b and the second photodetector 812b may include a photo diode, a photo transistor, or an image sensor (e.g., a charge-coupled device (CCD) a complementary metal-oxide semiconductor (CMOS), but are not limited thereto.

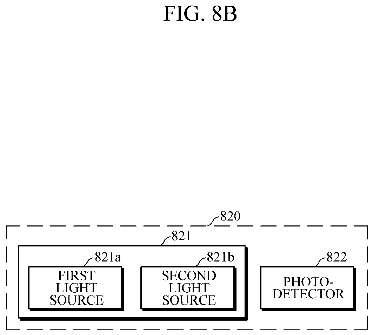

Referring to FIG. 8B, the pulse wave measurer 820 according to another embodiment may include a light source unit 821 including a plurality of light sources 821a and 821b and a photodetector 822. In FIG. 8B, two light sources are shown in the light source unit 821 for convenience of description, but the embodiment is not particularly limited to the number of light sources.

A first light source 821a emits light of a first wavelength to an object of interest and a second light source 821b emits light of a second wavelength to the object. Here, the first wavelength may be different from the second wavelength.

For example, the first light source 821a and the second light source 821b may be driven in a time-division manner according to the control of the processor and may sequentially or simultaneously emit light to the object of interest. In this case, light-source driving conditions, such as emission time, an order of driving, a current intensity, and pulse duration of each of the first light source 821a and the second light source 821b may be set in advance. The processor may control the driving of each light source 821a and 821b by referring to the light-source driving conditions.

The photodetector 822 may sequentially or simultaneously detect light of the first wavelength and light of second wavelength which return from the object of interest sequentially or simultaneously irradiated by the respective first and second light sources 821a and 821b and measure a first pulse wave signal and a second pulse wave signal.

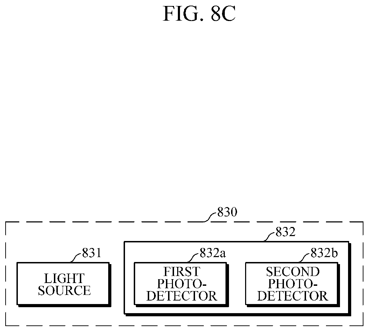

Referring to FIG. 8C, the pulse wave measurer 830 according to another embodiment may include a single light source 831 and a photodetector unit 832. The photodetector unit 832 may include a first photodetector 832a and a second photodetector 832b. However, FIG. 5C illustrates two photodetectors in the photodetector unit 832 for convenience of description, but the embodiment is not particularly limited to the number of photodetectors.

The single light source 831 may emit light of a specific wavelength to an object of interest. In this case, the single light source 831 may be formed to emit light of a wide wavelength range including visible wavelengths.

The photodetector unit 832 may receive light of the specific wavelength returning from the object and measure a plurality of pulse wave signals. To this end, the photodetector 832 may be formed to have a plurality of different response characteristics.

For example, the first photodetector 832a and the second photodetector 832 may be formed with photodiodes having different measurement ranges to respond to different wavelengths of light returning from the object of interest. Alternatively, one of the first photodetector 832a and the second photodetector 832b may have a color filter installed on the front surface thereof or the respective first and second photodetectors 832a and 832b may have different color filters installed on the front surfaces thereof to respond to different wavelengths of light. Alternatively, the first photodetector 832a and the second photodetector 832b may be disposed at different distances from the single light source 831. In this case, the photodetector disposed at a relatively short distance from the single light source 831 may detect light of a short wavelength band and the other photodetector disposed at a relatively far distance from the single light source 831 may emit light of a long wavelength band.

The examples of the pulse wave measurers to measure a plurality of pulse wave signals are described hereinabove with reference to FIGS. 8A to 8C. However, embodiments are not limited to the examples set forth herein, and the number and arrangement of the light sources and photodetectors may vary according to the application purpose of the pulse wave measurer and the size and shape of the electronic device in which the pulse wave measurer is mounted.

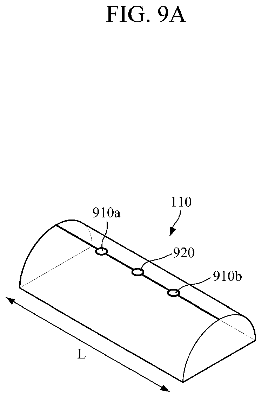

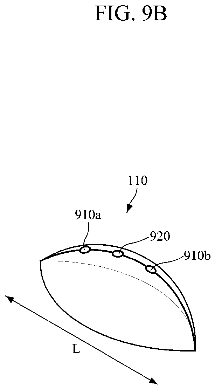

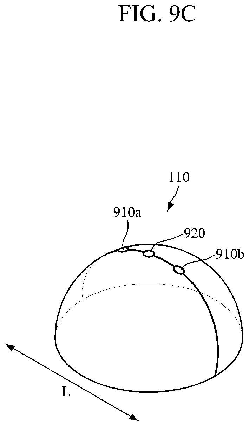

FIGS. 9A to 9C are diagrams illustrating examples of an arrangement of light sources and a photodetector. More specifically, FIG. 9A is a diagram illustrating an example of an arrangement of light sources and a photodetector when a pulse wave sensor is formed in a semi-cylindrical shape, FIG. 9B is a diagram illustrating an example of an arrangement of light sources and a photodetector when a pulse wave sensor is formed in a hemi-ellipsoid shape, and FIG. 9C is a diagram illustrating an example of an arrangement of a light source and a photodetector when a pulse wave sensor is formed in a hemispherical shape. FIGS. 9A to 9C illustrate two light sources 910a and 910b and one photodetector 920, but these are merely provided for convenience of description and the number of light sources and the photodetectors is not particularly limited.

Referring to FIGS. 9A to 9C, a pulse wave sensor 110 may include two light sources 910a and 910b and one photodetector 920.

The photodetector 920 may be disposed at the center of a curved surface, which is a contact surface, and the two light sources 910a and 910b may be arranged symmetrically with respect to the photodetector 920 along a length direction of the pulse wave sensor 110 or a tangential direction of the curved surface. In this case, the two light sources 910a and 910b may be disposed inside (e.g., 0.1 L to 0.9 L; L is a length of the pulse wave sensor) an edge portion in order to reduce the effect of the edge on the pressure or force.

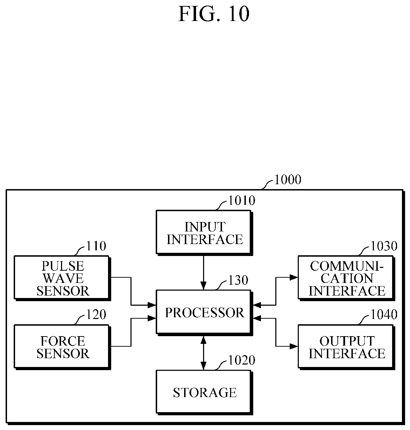

FIG. 10 is a diagram illustrating an apparatus for measuring bio-information according to another embodiment of the present disclosure. The apparatus 1000 for measuring bio-information of FIG. 10 may be mounted in an electronic device, an accessory of an electronic device (e.g., a protective case of an electronic device or the like), a stylus pen, or the like. In this case, the electronic device may include a mobile phone, a smartphone, a tablet terminal, a notebook computer, a PDA, a PMP, a navigation device, an MP3 player, a digital camera, a wearable device, and the like, and the wearable device may include various types of wearable devices, such as a wristwatch type, a wrist-band type, a belt type, a necklace type, an ankle band type, a thigh band type, a forearm band type, and the like. However, the electronic device is not limited to the above examples and the wearable device is also not limited to the above examples.

Referring to FIG. 10, the apparatus 1000 may include a pulse wave sensor 110, a force sensor 120, a processor 130, an input interface 1010, a storage 1020, a communication interface 1030, and an output interface 1040. In this case, the pulse wave sensor 110, the force sensor 120, and the processor 130 are similar to those described with reference to FIGS. 1 to 9C, and thus detailed descriptions thereof will be omitted.

The input interface 1010 may receive various operation signals input by a user. According to one embodiment, the input interface 1010 may include a key pad, a dome switch, a touchpad (resistive/capacitive) a jog wheel, a jog switch, a hardware button, and the like. In particular, when a touchpad has a layered structure with a display, this structure may be referred to as a touch screen.

Programs or instructions for operations of the apparatus 1000 for measuring bio-information may be stored in the storage 1020 and data input to and output from the apparatus 1000 may also be stored in the storage 1020. Also, the storage 1020 may store data processed by the apparatus 1000 and information required by the apparatus 1000 to process data.

The storage 1020 may include at least one type of storage medium, such as a flash memory, a hard disk type memory, a multimedia card micro type memory, a card-type memory (e.g., SD or XD memory), random access memory (RAM), static random access memory (SRAM), read only memory (ROM), electrically erasable programmable read only memory (EEPROM), programmable read only memory (PROM), magnetic memory, magnetic disk, and optical disk. In addition, the apparatus 1000 may operate an external storage medium, such as web storage providing a storage function of the storage 1020.

The communication interface 1030 may communicate with an external device. For example, the communication interface 1030 may transmit data handled by the apparatus 1000 or processing result data of the apparatus 1000 to the external device or may receive a variety of data necessary or useful to measure pulse wave signals and a contact force and/or estimate bio-information.

Here, the external device may be medical equipment which uses the data handled by the apparatus 1000 or the processing result data of the apparatus 1000, or may be a printer or display device to output a result. In addition, the external device may include, but not limited to, a digital TV, a desktop computer, a mobile phone, a smartphone, a tablet computer, a notebook computer, a PDA, a PMP, a navigation terminal, an MP3 player, a digital camera, a wearable device, and the like.

The communication interface 1030 may communicate with the external device using various communication technologies, such as Bluetooth, Bluetooth low energy (BLE), near field communication (NFC), wireless local area network (WLAN) communication, ZigBee communication, infrared data association (IrDA) communication, Wi-Fi direct (WFD) communication, ultra-wideband (UWB) communication, Ant+ communication, Wi-Fi communication, RFID communication, 3G communication, 4G communication, 5G communication, and the like. However, these are merely examples, and embodiments are not limited thereto.

The output interface 1040 may output the data handled by the apparatus 1000, the processing result data of the apparatus 1000, or the like. According to one embodiment, the output interface 1040 may output the data handled by the apparatus 1000, the processing result data of the apparatus 1000, or the like in at least one of visual, audible, and tactile manners. To this end, the output interface 1040 may include a display, a speaker, a vibrator, and the like.

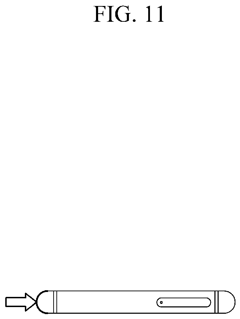

FIGS. 11 to 17 are diagrams illustrating examples of implementation of an apparatus for measuring bio-information.

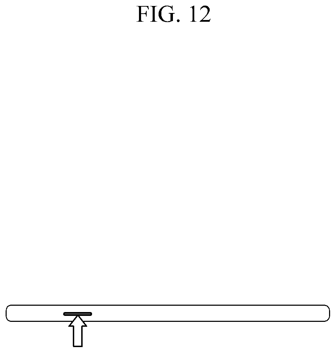

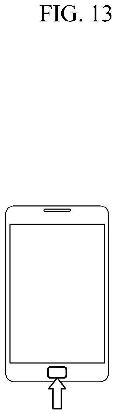

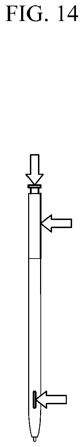

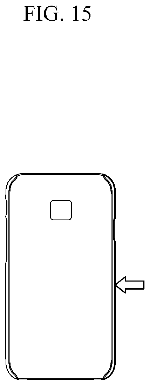

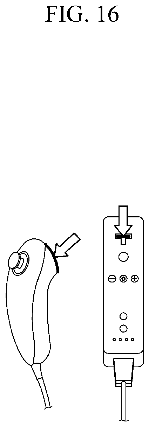

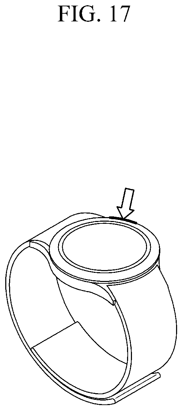

The apparatus 100 or 1000 for measuring bio-information may be applied to an edge of a smartphone (see FIG. 11), a side-button of a smartphone (see FIG. 12), a home-button of a smartphone (see FIG. 13), a button or frame on a stylus pen (see FIG. 14), an edge of a protective case of a smartphone (see FIG. 15), a button or edge of a joystick (see FIG. 16), and an edge or strap of a watch-type wearable device (see FIG. 17).

Meanwhile, FIGS. 11 to 17 are merely embodiments and the present disclosure is not limited thereto. That is, the apparatus 100 or 1000 may be applied without limitation as long as it is applied to a curved surface or a button of an electronic device, an accessory of an electronic device (e.g., a protective case of an electronic device), a stylus pen, a joystick, or the like.

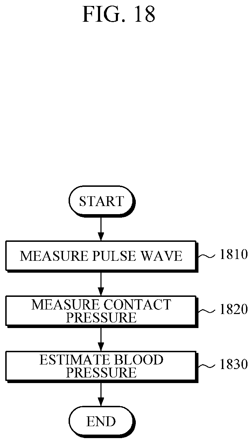

FIG. 18 is a flowchart illustrating a method of measuring bio-information according to one embodiment of the present disclosure. The method shown in FIG. 18 may be performed by the apparatuses 100 and 1000 of FIGS. 1 and 10 to measure bio-information.

Referring to FIG. 18, the apparatus for measuring bio-information may measure one or a plurality of pulse wave signals from an object of interest in contact with a circular contact surface of a pulse wave sensor (1810). In this case, the pulse wave signal may be a PPG signal. When the apparatus for measuring bio-information measures a plurality of pulse wave signals, the plurality of pulse wave signals may be measured using light of different wavelengths. According to one embodiment, the apparatus may emit light to the object of interest in contact with the circular contact surface of the apparatus and receive light returning from the object to measure one or more pulse wave signals.

The apparatus for measuring bio-information may measure a contact force between the object and the pulse wave sensor (1820). According to one embodiment, the apparatus for measuring bio-information may measure a force exerted on a force sensor disposed below or on a side of the pulse wave sensor due to the contact between the object and the pulse wave sensor.

The apparatus may estimate bio-information of the object of interest on the basis of the one or the plurality of measured pulse wave signals and the measured contact force (1830). The apparatus may acquire an oscillometric waveform using the pulse wave signals and the contact force. For example, the apparatus may select one or more pulse wave signals from among the one or the plurality of measured pulse wave signals according to predetermined criteria and calculate a contact pressure using the measured contact force. In addition, the apparatus may acquire the oscillometric waveform using a combination of the one or more selected pulse wave signals and the contact force. In this case, the predetermined criteria may include at least one of the maximum amplitude value of each of the pulse wave signals, an average amplitude value, and a difference between the maximum amplitude value and the minimum amplitude value of each of the pulse wave signals. However, the embodiment is not limited thereto and a pulse wave signal measured using light of a predetermined wavelength may be selected from among the acquired pulse wave signals. The apparatus may estimate the bio-information by analyzing the change in oscillometric waveform in accordance with the change in contact pressure. In this case, the bio-information may include, but not limited to, blood pressure, blood sugar level, cholesterol level, vascular age, arterial stiffness, aortic artery pressure waveform, stress index, fatigue level, and the like.

A blood pressure may include DBP, SBP, and MAP, and a contact pressure applied to the object of interest may act as an external pressure applied to a blood vessel. When the contact pressure is smaller than the MAP, the elastic restoring force of tissues acts in a direction of compressing the blood vessel and thereby the amplitude of the oscillometric waveform becomes smaller. When the contact pressure is equal to the MAP, the elastic restoring force of tissues becomes zero and thus does not affect the blood vessel so that the amplitude of the oscillometric waveform is maximum. In addition, when the contact pressure is greater than the MAP, the elastic restoring force of tissues acts in a direction of expanding the blood vessel, and thereby the amplitude of the oscillometric waveform becomes smaller. Accordingly, the apparatus for measuring bio-information may analyze the change in oscillometric waveform in accordance with the contact pressure and estimate the MAP using the contact pressure at a point where the amplitude of the oscillometric waveform is maximum. In addition, the apparatus for measuring bio-information may estimate the SBP using a contact pressure at a point where an amplitude is a first proportion (e.g., 0.6) relative to the maximum amplitude and estimate the DBP using a contact pressure at a point where an amplitude is a second proportion (e.g., 0.7) relative to the maximum amplitude.

For example, the apparatus for measuring bio-information may estimate the blood pressure using the measure contact force value and the above-described blood pressure calculation equations as shown in Equations 5 and 6.

Meanwhile, operation 1810 of measuring the pulse wave signals and operation 1820 of measuring the contact force may not be performed in the order described above and may be performed simultaneously for a predetermined length of time.

According to an additional embodiment, the apparatus may generate contact pressure guide information on the basis of the calculated contact pressure during the measurement of the pulse wave signals and provide the generated contact pressure guide information to the user.

The current embodiments can be implemented as computer readable codes in a computer readable record medium. Codes and code segments constituting the computer program can be easily inferred by a skilled computer programmer in the art. The computer readable record medium includes all types of record media in which computer readable data are stored. Examples of the computer readable record medium include a ROM, a RAM, a CD-ROM, a magnetic tape, a floppy disk, and an optical data storage. Further, the record medium may be implemented in the form of a carrier wave such as Internet transmission. In addition, the computer readable record medium may be distributed to computer systems over a network, in which computer readable codes may be stored and executed in a distributed manner.

**Claims**

1. An apparatus (100) for measuring bio-information, comprising:

   a pulse wave sensor (110) comprising a housing (111) with a curved contact surface, convex toward a contact surface of an object of interest, and configured to measure (1810) one or more pulse wave signals from the object when in contact with the contact surface;
   a force sensor (120) disposed below or on a side of the pulse wave sensor and configured to measure (1820) a contact force of the object; and
   a processor (130) configured to estimate (1830) bio-information of the object on the basis of the one or more measured pulse wave signals and the measured contact force,
   **characterized in that**
   the housing (111) is formed in a semi-cylindrical shape or a hemi-ellipsoid shape having the following dimensions:

   a first radius of curvature R1, defining a maximum width of the housing (111) in a width direction of the pulse wave sensor (110), which is not less than 2 mm and not more than 10 mm,
   a length L, defining a maximum length of the housing (111) in a length direction of the pulse wave sensor (110) that is perpendicular to the width direction, which is less than or equal to 16 mm, and
   a second radius of curvature R2, defining a maximum height of the housing (111) in a height direction of the pulse wave sensor (110) that is perpendicular to the width direction and length direction, which is not less than 0.5 * R1 and not more than 4 * R1.

2. The apparatus (100) of claim 1, wherein the pulse wave sensor (110) further comprises a pulse wave measurer (112; 810; 820; 830) mounted in the housing and configured to measure (1810) the one or more pulse wave signals from the object in contact with the contact surface.

3. The apparatus (100) of claim 2,

   wherein the housing has a surface roughness of 1.6 $\mu$m or less, or
   wherein the housing has a stiffness of 0.5 GPa or greater, or
   wherein the pulse wave signals are photoplethysmogram, PPG, signals, or
   wherein the pulse wave measurer (112; 810; 820; 830) comprises one or more light sources (811a, 812a; 821a, 821b; 831) configured to emit light to the object of interest and a photodetector (811b, 812b; 822; 832a, 832b) configured to receive light returning from the object and measure the one or more pulse wave signals.

4. The apparatus (100) of any one of the preceding claims, wherein the processor (130) is further configured to acquire an oscillometric waveform using the one or more pulse wave signals and the contact force and estimates the bio-information by analyzing a change in oscillometric waveform.

5. The apparatus (100) of any claim 4, wherein the processor (130) is further configured to select one or more pulse wave signals from among the one or more pulse wave signals and acquire the oscillometric waveform using the one or more selected pulse wave signals and the contact force.

6. The apparatus (100) of claim 5, wherein the processor is further configured to select the one or more pulse wave signals on the basis of at least one of a maximum amplitude value of each of the pulse wave signals, an average amplitude value, and a difference between a maximum amplitude value and a minimum amplitude value of each of the pulse wave signals.

7. The apparatus (100) of any one of the preceding claims, wherein the processor is further configured to generate and provide contact pressure guide information on the basis of the measured contact force during the measurement of the plurality of pulse wave signals, or wherein the bio-information is blood pressure.

8. The apparatus (100) of any one of the preceding claims, wherein the apparatus does not include a contact area sensor that measures a contact area with the object of interest.

**Patentansprüche**

1. Eine Vorrichtung (100) zur Messung von Bio-Informationen, die Folgendes umfasst:

   einen Pulswellensensor (110), der ein Gehäuse (111) mit einer gekrümmten Kontaktfläche umfasst, die zu einer Kontaktfläche eines interessierenden Objekts hin konvex ist, und der so konfiguriert ist, dass er ein oder mehrere Pulswellensignale von dem Objekt misst (1810), wenn es in Kontakt mit der Kontaktfläche ist;
   einen Kraftsensor (120), der unter oder auf einer Seite des Pulswellensensors angeordnet und zum Messen (1820) einer Kontaktkraft des Objekts konfiguriert ist; und
   einen Prozessor (130), der so konfiguriert ist, dass er Bio-Informationen des Objekts auf der Grundlage des einen oder der mehreren gemessenen Pulswellensignale und der gemessenen Kontaktkraft schätzt (1830), **dadurch gekennzeichnet, dass** das Gehäuse (111) eine halbzylindrische oder halbellipsoide Form mit den folgenden Abmessungen aufweist:

   einen ersten Krümmungsradius R1, der eine maximale Breite des Gehäuses (111) in einer Breitenrichtung des Pulswellensensors (110) definiert, die nicht weniger als 2 mm und nicht mehr als 10 mm beträgt,
   eine Länge L, die eine maximale Länge des Gehäuses (111) in einer Längenrichtung des Pulswellensensors (110) definiert, die senkrecht zur Breitenrichtung ist, die kleiner oder gleich 16 mm ist, und
   einen zweiten Krümmungsradius R2, der eine maximale Höhe des Gehäuses (111) in einer Höhenrichtung des Pulswellensensors (110) definiert, die senkrecht zu der Breitenrichtung und der Längenrichtung ist, die nicht weniger als 0,5 * R1 und nicht mehr als 4 * R1 beträgt.

2. Die Vorrichtung (100) nach Anspruch 1, wobei der Pulswellensensor (110) ferner einen Pulswellenmesser (112; 810; 820; 830) umfasst, der in dem Gehäuse angebracht und so konfiguriert ist, dass er das eine oder die mehreren Pulswellensignale von dem mit der Kontaktfläche in Kontakt stehenden Objekt misst (1810).

3. Die Vorrichtung (100) nach Anspruch 2,

   wobei das Gehäuse eine Oberflächenrauhigkeit von 1,6 μm oder weniger aufweist, oder
   wobei das Gehäuse eine Steifigkeit von 0,5 GPa oder mehr aufweist, oder
   wobei die Pulswellensignale Photoplethysmogramm-, PPG-, Signale sind, oder
   wobei das Pulswellenmessgerät (112; 810; 820; 830) eine oder mehrere Lichtquellen (811a, 812a, 821a, 821b; 831), die so konfiguriert sind, dass sie Licht zu dem interessierenden Objekt emittieren, und einen Fotodetektor (811b, 812b; 822; 832a, 832b) umfasst, der so konfiguriert ist, dass er das von dem Objekt zurückkehrende Licht empfängt und die ein oder mehreren Pulswellensignale misst.

4. Die Vorrichtung (100) nach einem der vorhergehenden Ansprüche, wobei der Prozessor (130) ferner so konfiguriert ist, dass er eine oszillometrische Wellenform unter Verwendung des einen oder der mehreren Pulswellensignale und der Kontaktkraft erfasst und die Bio-Information durch Analysieren einer Änderung der oszillometrischen Wellenform schätzt.

5. Die Vorrichtung (100) nach einem der Ansprüche 4, wobei der Prozessor (130) ferner so konfiguriert ist, dass er ein oder mehrere Pulswellensignale aus dem einen oder den mehreren Pulswellensignalen auswählt und die oszillometrische Wellenform unter Verwendung des einen oder der mehreren ausgewählten Pulswellensignale und der Kontaktkraft erfasst.

6. Die Vorrichtung (100) nach Anspruch 5, wobei der Prozessor ferner so konfiguriert ist, dass er das eine oder die mehreren Pulswellensignale auf der Grundlage von mindestens einem der folgenden Werte auswählt: einem maximalen Amplitudenwert jedes der Pulswellensignale, einem durchschnittlichen Amplitudenwert und einer Differenz zwischen einem maximalen Amplitudenwert und einem minimalen Amplitudenwert jedes der Pulswellensignale.

7. Die Vorrichtung (100) nach einem der vorhergehenden Ansprüche, wobei der Prozessor ferner so konfiguriert ist, dass er auf der Grundlage der gemessenen Kontaktkraft während der Messung der mehreren Pulswellensignale Kontaktdruckführungsinformationen erzeugt und bereitstellt, oder
   wobei die Bio-Information der Blutdruck ist.

8. Die Vorrichtung (100) nach einem der vorhergehenden Ansprüche, wobei die Vorrichtung keinen Kontaktflächensensor enthält, der eine Kontaktfläche mit dem Objekt von Interesse misst.

**Revendications**

1. Appareil (100) pour mesurer des informations biologiques, « bio-informations », comprenant :

   un capteur d'onde d'impulsion (110) comprenant un boîtier (111) avec une surface de contact incurvée, convexe vers une surface de contact d'un objet d'intérêt, et configuré pour mesurer (1810) un ou plusieurs signaux d'onde d'impulsion issus de l'objet lorsqu'en contact avec la surface de contact ;
   un capteur de force (120) disposé au-dessous ou sur un côté du capteur d'onde d'impulsion et configuré pour mesurer (1820) une force de contact de l'objet ; et
   un processeur (130) configuré pour estimer (1830) des bio-informations de l'objet en s'appuyant sur l'un ou plusieurs signaux d'onde d'impulsion mesurés et la force de contact mesurée,
   **caractérisé en ce que**
   le boîtier (111) est constitué dans une forme semicylindrique ou une forme hémi-ellipsoïde présentant les dimensions suivantes :

   un premier rayon de courbure R1, définissant une largeur maximale du boîtier (111) dans une direction de largeur du capteur d'onde d'impulsion (110), qui n'est ni inférieur à 2 mm, ni supérieur à 10 mm,
   une longueur L, définissant une longueur maximale du boîtier (111) dans une direction de longueur du capteur d'onde d'impulsion (110) qui est perpendiculaire à la direction de largeur, qui est inférieure ou égale à 16 mm, et
   un deuxième rayon de courbure R2, définissant une hauteur maximale du boîtier (111) dans une direction de hauteur du capteur d'onde d'impulsion (110), qui est perpendiculaire à la direction de largeur et la direction de longueur, qui n'est ni inférieure à 0,5 x R1, ni supérieure à 4 x R1.

2. L'appareil (100) de la revendication 1, dans lequel le capteur d'onde d'impulsion (110) comprend en outre un mesureur d'onde d'impulsion (112 ; 810 ; 820 ; 830) monté dans le boîtier et configuré pour mesurer (1810) l'un ou plusieurs signaux d'onde d'impulsion issus de l'objet en contact avec la surface de contact.

3. L'appareil (100) de la revendication 2,

   dans lequel le boîtier présente une rugosité de surface de 1,6 $\mu$m ou moins, ou
   dans lequel le boîtier présente une rigidité de 0,5 Gpa ou plus, ou
   dans lequel les signaux d'onde d'impulsion sont des signaux de type photopléthysmogramme, PPG, ou
   dans lequel le mesureur d'onde d'impulsion (112 ; 810 ; 820 ; 830) comprend une ou plusieurs sources de lumière (811a, 812a ; 821a, 821b ; 831) configurées pour émettre une lumière vers l'objet d'intérêt, et un photodétecteur (811b, 812b ; 822 ; 832a, 832b) configuré pour recevoir une lumière revenant de l'objet et mesurer l'un ou plusieurs signaux d'onde d'impulsion.

4. L'appareil (100) de l'une quelconque des revendications précédentes, dans lequel le processeur (130) est configuré en outre pour acquérir une forme d'onde oscillométrique en utilisant l'un ou plusieurs signaux d'onde d'impulsion et la force de contact, et estime les bio-informations en analysant un changement dans une forme d'onde oscillométrique.

5. L'appareil (100) de la revendication 4, dans lequel le processeur (130) est configuré en outre pour sélectionner un ou plusieurs signaux d'onde d'impulsion parmi l'un ou plusieurs signaux d'onde d'impulsion, et acquérir la forme d'onde oscillométrique en utilisant l'un ou plusieurs signaux d'onde d'impulsion sélectionnés et la force de contact.

6. L'appareil (100) de la revendication 5, dans lequel le processeur est configuré en outre pour sélectionner l'un ou plusieurs signaux d'onde d'impulsion en s'appuyant sur au moins une d'une valeur d'amplitude maximale de chacun des signaux d'onde d'impulsion, d'une valeur d'amplitude moyenne et d'une différence entre une valeur d'amplitude maximale et une valeur d'amplitude minimale de chacun des signaux d'onde d'impulsion.

7. L'appareil (100) de l'une quelconque des revendications précédentes, dans lequel le processeur est configuré en outre pour générer et fournir des informations de guidage de pression de contact en s'appuyant sur la force de contact mesurée au cours de la mesure de la pluralité de signaux d'onde d'impulsion, ou
   dans lequel les bio-informations consistent en une pression artérielle.

8. L'appareil (100) de l'une quelconque des revendications précédentes, dans lequel l'appareil n'inclut pas un capteur de zone de contact qui mesure une zone de contact avec l'objet d'intérêt.

FIG. 1

FIG. 2

FIG. 3

FIG. 4

FIG. 5

FIG. 6A

FIG. 6B

FIG. 7A

## FIG. 7B

FIG. 7C

FIG. 7D

SBP

740

730

CONTACT FORCE

# FIG. 8A

FIG. 8B

FIG. 8C

FIG. 9A

FIG. 9B

## FIG. 9C

FIG. 10

FIG. 11

FIG. 12

FIG. 13

FIG. 14

FIG. 15

FIG. 16

FIG. 17

FIG. 18

```
        ( START )
            │
            ▼
┌──────────────────────┐
│  MEASURE PULSE WAVE  │ ～ 1810
└──────────────────────┘
            │
            ▼
┌──────────────────────┐
│   MEASURE CONTACT    │ ～ 1820
│      PRESSURE        │
└──────────────────────┘
            │
            ▼
┌──────────────────────┐
│   ESTIMATE BLOOD     │ ～ 1830
│      PRESSURE        │
└──────────────────────┘
            │
            ▼
        (  END  )
```

## REFERENCES CITED IN THE DESCRIPTION

**Patent documents cited in the description**

- WO 2009125349 A2

- US 2017127958 A1

**Non-patent literature cited in the description**

- The effect of contacting force on photoplethysmorgraphic signals; The effect of contacting force on photoplethysmorgraphic signals. **TENG X F et al.** Physiological Measurement. Institute of Physics Publishing, 01 October 2004, vol. 25